# EUROPÄISCHE PATENTANMELDUNG

(11) **EP 1 634 867 A1**
(43) Veröffentlichungstag der Anmeldung: **15.03.2006**
(21) Anmeldenummer: 04020116.2
(22) Anmeldetag: 25.08.2004
(51) Int. Cl.: C07C 255/05, C07D 233/56, C07D 213/18

(54) **Tricyanomethansalze organischer Kationen**

(71) Anmelder: Lonza AG, 4052 Basel (CH)
(72) Erfinder: Taeschler, Christoph, 3912 Termen (CH)

(57) **Zusammenfassung**

Die Erfindung betrifft Tricyanomethansalze der Formel 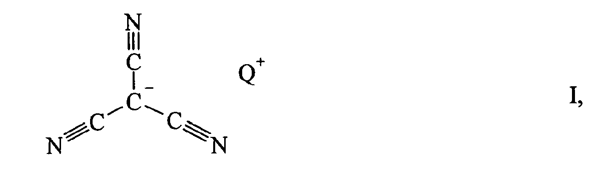 worin Q⁺ ein organisches Kation ist, das mindestens ein Heteroatom enthält, ausgewählt aus der Gruppe bestehend aus Stickstoff, Phosphor, Schwefel und Sauerstoff, deren Verwendung, sowie ein Verfahren zu deren Herstellung.

## Beschreibung

Die Erfindung betrifft Tricyanomethansalze der Formel 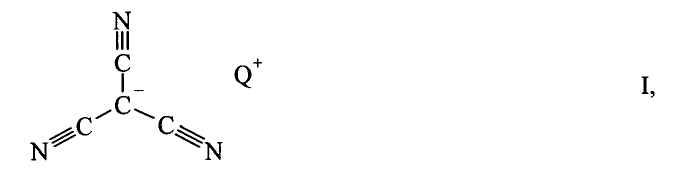
worin Q⁺ ein organisches Kation ist, das mindestens ein Heteroatom enthält, ausgewählt aus der Gruppe bestehend aus Stickstoff, Phosphor, Schwefel und Sauerstoff, deren Verwendung, sowie ein Verfahren zu deren Herstellung.

Tricyanomethanmetallsalze und Verfahren zu deren Herstellung sind bekannt aus Schmidtmann, H., *Ber.* 29, **1896,** 1172 und Trofimenko, S. et al., *J*. *Org. Chem.* 27, **1962,** 433-438. Aus Batten, S.R. et al., *Chem. Commun.* **1998,** 439-440 sind ferner Tricyanomethanmetallsalzkomplexe mit organischen Liganden bekannt. Die erfindungsgemässen Tricyanomethansalze organischer Kationen sind jedoch bislang nicht beschrieben worden.

Salze organischer Kationen weisen einen niedrigen Dampfdruck sowie oft einen niedrigen Schmelzpunkt auf und eignen sich durch ihre physikalischen Eigenschaften für die Verwendung als Lösungsmittel. Sie werden dann als ionischen Flüssigkeiten bezeichnet und sind in den letzten Jahren als umweltfreundliche Lösungsmittel für eine "grüne Chemie" bekannt geworden. In Abhängigkeit von den gewählten Anionen und Kationen lässt sich der Schmelzpunkt über einen weiten Temperaturbereich variieren, so dass ionische Flüssigkeiten für einen Anwendungsbereich von -60 °C bis über 300 °C zur Verfügung stehen.

Üblicherweise sind die organischen Kationen ionischer Flüssigkeiten einwertige quartäre Ammonium- oder Phosphoniumbasen, oder Kationen von aromatischen, meist stickstoffhaltigen Basen, welche gegebenenfalls zusätzlich mit Alkylgruppen, Halogenatomen oder Cyanogruppen substituiert sind und weitere Heteroatome wie Phosphor, Schwefel oder Sauerstoff enthalten können. Beispiele für gängige organische Kationen sind Imidazolium-, Oxazolium-, Pyrazinium-, Pyrazolium-, Pyridazinium-, Pyrrolidinium-, Pyrimidinium-, Thiazolium- und Triazoliumkationen. Imidazolium- und Pyrrolidiniumkationen werden am häufigsten verwendet. Üblicherweise werden die Kationen mit einer positiven Formalladung lokalisiert auf dem Heteroatom, delokalisiert in der Mitte eines Ringes oder auch als Summenladung in Komplexschreibweise dargestellt. Alle Darstellungsweisen werden dabei äquivalent verwendet.

Typische Anionen in ionischen Flüssigkeiten sind Acetat, AlCl₄⁻, AsF₆⁻ , BF₄⁻, Bromid, CF₃SO₃⁻, (CF₃)₂PF₄⁻, (CF₃)₃PF₃⁻, (CF₃)₄PF₂⁻, (CF₃)₅PF⁻, (CF₃)₆P⁻, Chlorid, CN⁻, FeCl₃⁻, NO₃⁻, PF₆⁻, Pyruvat, Trifluormethansulfonat, Oxalat oder SCN⁻. Am häufigsten werden AlCl₄⁻, AsF₆⁻, BF₄⁻ und PF₆⁻ verwendet.

Ausgangsmaterialien für die Herstellung von ionischen Flüssigkeiten können beispielsweise von Merck KGaA, Darmstadt oder IoLiTec, A. Bösmann, Dr. T. Schubert G.b.R., Freiburg bezogen werden.

Aufgabe der vorliegenden Erfindung war es, neue ionische Flüssigkeiten und kostengünstige Verfahren zu deren Herstellung zur Verfügung zu stellen. Diese Verbindungen sollten nach Gebrauch umweltschonend entsorgt werden können.

Diese Aufgabe wird entsprechend Anspruch 1 gelöst.

Beansprucht werden Tricyanomethansalze der Formel 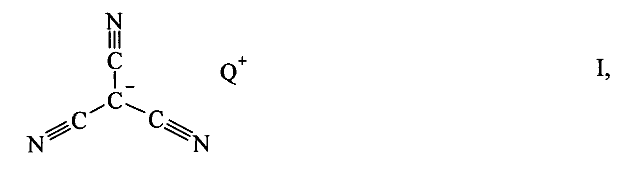
worin Q⁺ ein organisches Kation ist, das mindestens ein Heteroatom enthält, ausgewählt aus der Gruppe bestehend aus Stickstoff, Phosphor, Schwefel und Sauerstoff.

Die erfindungsgemässen Verbindungen auf Basis von Tricyanomethananidionen sind relativ unpolare ionische Flüssigkeiten, die im Vergleich mit anderen ionischen Flüssigkeiten niedrige Wasserlöslichkeiten und niedrige Viskositäten aufweisen.

Die Verwendung der erfindungsgemässen Verbindungen auf Basis von Tricyanomethananidionen hat gegenüber vielen anderen ionischen Flüssigkeiten den Vorteil, dass bei der thermischen Entsorgung keine metallhaltigen Rückstände entstehen.

Das erfindungsgemässe Verfahren erlaubt die Erstellung von ionischen Flüssigkeiten mit einem sehr niedrigen Chloridgehalt.

Die erfindungsgemässen Tricyanomethanverbindungen lassen sich auch aus den in Trofimenko et al. genannten Tricyanomethanmetallsalzkomplexen herstellen, was aber sehr aufwendig und kostspielig ist. Die Synthese dieser Metallkomplexe beginnt mit einem Dihalomalonsäuredinitril, das zu einem Tricyanomethanhalogenid umgesetzt wird.

Ein Vorteil des erfindungsgemässen Verfahrens ist die Möglichkeit der Bereitstellung von weitgehend chlorid- und natriumfreien ionischen Flüssigkeiten.

Vorzugsweise wird das organische Kation Q⁺ ausgewählt aus der Gruppe bestehend aus Kationen der Formel
a) [WR¹R²R³R⁴]⁺, worin W ein Stickstoff- oder Phosphoratom bedeutet, und
   i) worin R¹ bis R³ unabhängig voneinander C₁₋₂₀-Alkyl sind, und R⁴ C₁₋₂₀-Alkyl, C₃₋₁₀-Cycloalkyl oder C₆₋₁₀-Aryl ist, wobei R¹ bis R⁴ unabhängig voneinander gegebenenfalls ein oder mehrere Halogenatome enthalten, oder
   ii) worin R¹ und R² zusammen einen 5- bis 7-gliedrigen Ring bilden, und worin R³ und R⁴ unabhängig voneinander C₁₋₂₀-Alkyl sind, wobei R³ und R⁴ unabhängig voneinander gegebenenfalls ein oder mehrere Halogenatome enthalten, oder
   iii) worin R¹ und R², sowie R³ und R⁴ jeweils einen 5- bis 7-gliedrigen Ring bilden, oder
b) [XR⁵R⁶R⁷]⁺, worin X ein Stickstoffatom bedeutet, und worin R⁵ und R⁶ gemeinsam mit X einen Ring bilden, in welchem X formal eine Einfach- und eine Doppelbindung aufweist, und R⁷ C₁₋₂₀-Alkyl, C₃₋₁₀-CYcloalkyl oder C₆₋₁₀-Aryl ist, wobei R⁷ gegebenenfalls ein oder mehrere Halogenatome enthält, oder
c) [YR⁸R⁹R¹⁰]⁺, worin Y ein Schwefelatom bedeutet, und
   i) worin R⁸ und R⁹ unabhängig voneinander C₁₋₂₀-Alkyl sind, und R¹⁰ C₁₋₂₀-Alkyl, C₃₋₁₀-Cycloalkyl oder C₆₋₁₀-Aryl ist, wobei R⁸ bis R¹⁰ unabhängig voneinander gegebenenfalls ein oder mehrere Halogenatome enthalten, oder
   ii) worin R⁸ und R⁹ gemeinsam einen 5- bis 7-gliedrigen Ring bilden, und R¹⁰ C₁₋₂₀-Alkyl, C₃₋₁₀-Cycloalkyl oder C₆₋₁₀-Aryl ist, wobei R¹⁰ gegebenenfalls ein oder mehrere Halogenatome enthält, oder
d) [ZR¹¹R¹²]⁺, worin Z ein Schwefel- oder Sauerstoffatom bedeutet, und worin R¹¹ und R¹² gemeinsam mit Z einen Ring bilden, in welchem Z formal eine Einfach- und eine Doppelbindung zu R¹¹ und R¹² aufweist, und
worin an jeden der von R¹ bis R¹² gebildeten Ringe gegebenenfalls ein oder mehrere Substituenten aus der Gruppe bestehend aus C₁₋₂₀-Alkylgruppen,
C₁₋₂₀-Alkoxygruppen, Halogen und Cyan gebunden sind, wobei die
C₁₋₂₀-Alkylgruppen und C₁₋₂₀-Alkoxygruppen unabhängig voneinader gegebenenfalls ein oder mehrere Halogenatome enthalten, und
worin jeder der von R¹ bis R¹² gebildeten Ringe gegebenenfalls ein oder zwei weitere Heteroatome aus der Gruppe bestehend aus Stickstoff, Schwefel und Sauerstoff enthält, und/oder an einen anderen aromatischen oder nichtaromatischen 5- bis 7-gliedrigen Ring anelliert ist.

Hier und im Folgenden bedeutet der Ausdruck "C₁₋ₙ-Alkyl" eine unverzweigte oder verzweigte Alkylgruppe mit 1 bis n Kohlenstoffatomen. C₁₋₂₀-Alkyl repräsentiert beispielsweise Methyl, Ethyl, Propyl, Isopropyl, Butyl, Isobutyl, *sec*-Butyl, *tert*-Butyl, Pentyl, 1,4-Dimethyl-pentyl, Hexyl, Heptyl, Octyl, 1,5-Dimethyl-hexyl, Nonyl, Decyl, 4-Ethyl-1,5-dimethylhexyl, Undecyl, Dodecyl, Tridecyl, Tetradecyl oder Eicosyl.

Hier und im Folgenden bedeutet der Ausdruck "C₃₋ₙ-Cycloalkyl" eine Cycloalkylgruppe mit 3 bis n Kohlenstoffatomen. C₃₋₁₀-Cycloalkyl repräsentiert beispielsweise Cyclopropyl, Cyclobutyl, Cyclobutyl, Cyclopentyl, Cyclohexyl, Cycloheptyl, Cyclooctyl, Cyclononyl oder Cyclodecyl.

Hier und im Folgenden bedeutet der Ausdruck "C₆₋₁₀-Aryl" eine Arylgruppe mit 6 bis 10 Kohlenstoffatomen. C₆₋₁₀-Aryl repräsentiert beispielsweise Phenyl, Benzyl, Methylphenyl, Dimethylphenyl, Ethylmethylphenyl, Diehtylphenyl oder Naphthyl.

Hier und im Folgenden bedeutet der Ausdruck "C₁₋ₙ-Alkoxy" eine unverzweigte oder verzweigte Alkoxygruppe mit 1 bis n Kohlenstoffatomen. C₁₋₂₀-Alkoxy repräsentiert beispielsweise Methoxy, Ethoxy, Propoxy, Isopropoxy, Butoxy, Isobutoxy, *sec*-Butoxy, *tert*-Butoxy, Pentyloxy, 1,4-Dimethyl-pentyloxy, Hexyloxy, Heptyloxy, Octyloxy, 1,5-Dimethyl-hexyloxy, Nonyloxy, Decyloxy, 4-Ethyl-1,5-dimethylhexyloxy, Undecyloxy, Dodecyloxy, Tridecyloxy, Tetradecyloxy oder Eicosyloxy.

Hier und im Folgenden bedeutet der Ausdruck "Halogen" Fluor, Chlor, Brom oder Iod.

In einer bevorzugten Ausführungsform enthält das Kation Q⁺ mindestens einen 5- bis 7-gliedrigen aromatischen oder nichtaromatischen Ring.

In einer weiteren bevorzugten Ausführungsform enthält ein aromatischer oder nicht aromatischer Ring im organischen Kation Q⁺ ein bis zwei weitere Heteroatome aus der Gruppe bestehend aus Stickstoff, Schwefel oder Sauerstoff. Besonders bevorzugt enthält das Kation Q⁺ ein weiteres Stickstoff- oder Schwefelatom.
Weiterhin bevorzugt können an einen aromatischen oder nichtaromatischen Ring des Kations Q⁺ eine oder mehrere Substituenten aus der Gruppe bestehend aus C₁₋₂₀-Alkylgruppen, C₁₋₂₀-Alkoxygruppen, C₃₋₁₀-Cycloalkylgruppen, C₆₋₁₀-Arylgruppen, Halogen und Cyan gebunden sein. Hierbei enthalten die C₁₋₂₀-Alkylgruppen, C₁₋₂₀-Alkoxygruppen, C₃₋₁₀-Cycloalkylgruppen, C₆₋₁₀-Arylgruppen unabhängig voneinander gegebenenfalls ein oder mehrere Halogenatome.

Besonders bevorzugt wird das Kation Q⁺ ausgewählt aus der Gruppe bestehend aus organischen Ammonium-, Phosphonium- und Sulfoniumkationen, Pyrrolidinium-, Pyrrolinium-, Pyrazolium-, Pyrazolium, Imidazolium-, Triazolium-, Oxazolium-, Thiazolium-, Piperidinium-, Piperazinium-, Morpholinium-, Pyridinium-, Pyridazinium-, Pyrimidinium-, Pyrazinium-, 1,3-Dioxolium-, Pyrilium-, Thiopyrilium-, Azoniaspiro- und Phosphoniaspirokationen.

In einer weiteren besonders bevorzugten Ausführungsform wird das Kation Q⁺ ausgewählt aus der Gruppe bestehend aus *N,N-*Di(C₁₋₂₀-alkyl)-*N*,*N*-(dimethyl)ammonium-, *N*-(C₆₋₁₀-aryl)-*N,N,N*-Tri(C₁₋₂₀-Alkyl)-arnmonium-, *N,N*-Di(C₆₋₁₀-aryl)-*N,N*-(dimethyl)-ammonium-, Tetramethylphosphonium-, Tetrabutylphosphonium-, Trimethylsulfonium-, Tributylsulfonium, 1-(C₁₋₂₀-Alkyl)-3-methylpyridinium-, 1-(C₁₋₂₀-Alkyl)-3-cyanopyridinium-, 1-(C₁₋₂₀-Alkyl)-1-methylpyrrolidinium-, 1-(C₁₋₂₀-Alkyl)-1,3 -dimethylpiperidinium-, 1-(C₁₋₂₀-Alkyl)-1-methylimidazolium-, 1-(C₁₋₂₀-Alkyl)-3-ethylpyridinium-, 4,5-Dimethyl-2-(diphenylmethyl)-1,3-dioxolium-, 2-(Diphenylmethyl)-4,5-diphenyl-1,3-dioxolium, 6-azoniaspiro[5.5]undecan- und 5-Phosphoniaspiro[4.4]nonankationen.

Beansprucht werden im Besonderen die Verbindungen 1-Butyl-3-methylpyridiniumtricyanomethan, 1-Butyl-1-methylpyrrolidiniumtricyanomethan, 1-Butyl-1,3-dimethylpiperidiniumtricyanomethan, 1-Butyl-1-methylimidazoliumtricyanomethan, 1-Ethyl-3-methyl-imidazoliumtricyanomethan, Trimethylsulfoniumtricyanomethan, Tributylsulfoniumtricyanomethan, Tetrabutylphosphoniumtricyanomethan und Tetrabutylammoniumtricyanomethan.

Die erfmdungsgemässen Verbindungen der Formel I, worin Q⁺ wie oben definiert ist, sind ionische Flüssigkeiten und können, gegebenenfalls in einer Mischung mit einer oder mehreren anderen ionischen Flüssigkeiten, Wasser oder organischen Lösungsmitteln, als Lösungsmittel verwendet werden.

Die Polarität der erfindungsgemässen Verbindungen ist niedriger als bei üblichen ionischen Flüssigkeiten, jedoch höher als bei gewöhnlichen organischen Lösungsmitteln, so dass sich hier neue Anwendungsbereiche ergeben.

Die erfindungsgemässen Tricyanomethansalze können besonders bevorzugt verwendet werden beispielsweise als Bestandteil von polaren Lösungsmitteln, als Elektrolyte in der Elektrolyse oder in elektrischen Bauteilen, weiterhin für die Herstellung von Flüssigkristallen für LCD's oder von Leitgelen, letztere werden beispielsweise in der Photovoltaik verwendet.

Beansprucht wird ein Verfahren zur Herstellung der Tricyanomethansalze der Formel I, wobei Q⁺ wie oben definiert ist, umfassend:
a) Mischen von Malonsäuredinitril mit einer Cyanoverbindung der Formel RCN, worin R ausgewählt ist aus der Gruppe bestehend aus Chlor, Brom, Iod und Cyan in Gegenwart einer Base, gegebenenfalls in Gegenwart eines Lösungsmittels,
b) Zugabe eines Salzes (Q⁺)ₙX⁻ⁿ mit n = 1 oder 2, wobei Q⁺ wie oben definiert und X⁻ⁿ ein Anion ausgewählt aus der Gruppe bestehend aus Halogeniden, Pseudohalogeniden, Sulfat und organischen Säureanionen ist, und
worin das Anion X⁻ⁿ in wässriger Phase gegen ein aus Malonsäuredinitril und RCN *in situ* gebildetes Tricyanomethanidanion ausgetauscht wird.

In einer bevorzugten Verfahrensvariante wird Malonsäuredinitril, die Cyanoverbindung der Formel RCN und das Salz (Q⁺)ₙX⁻ⁿ in Gegenwart einer Base, gegebenenfalls in Gegenwart eines weiteren Lösungsmittels, in wässriger Phase gemischt, wobei Bildung und Austausch des Tricyanomethanidanions in einer Eintopfreaktion stattfinden.

Eine bestimmte Reihenfolge der Zugabe von Malonsäuredinitril, der Cyanoverbindung der Formel RCN, der Base, und des Salzes der Formel (Q⁺)ₙX⁻ⁿ ist nicht zwingend vorgeschrieben. Die Zugabereihenfolge in den Beispielen hat sich im prozesstechnischen Ablauf bewährt, im Labor führt jedoch jede andere Reihenfolge ebenfalls zur Bildung der erfindungsgemässen Verbindungen.

Sowohl die Cyanoverbindung der Formel RCN, als auch die Base und/oder das Salz der Formel (Q⁺)ₙX⁻ⁿ, wobei R, Q⁺, X⁻ⁿ und n wie oben definiert sind, können in reiner Form, wie auch als Lösung, beispielsweise in Wasser und/oder Methanol, verwendet werden.

Weitere geeignete Lösungsmittel und/oder Lösungsvermittler können ausgewählt werden aus der Gruppe bestehend aus Aceton, Acetonitril, C₁₋₄-Alkohole, Chloroform, Dichlorethan, Diethylether, DMSO, Ethylacetat, Hexan, Methylenchlorid, Propylencarbonat, Schwefelkohlenstoff, THF, Toluol, Xylol oder geeigneten Gemischen davon. Vorzugsweise werden wasserlösliche Lösungsmittel verwendet.

In einer bevorzugten Ausführungsform ist die Cyanoverbindung RCN ausgewählt aus der Gruppe bestehend aus Chlorcyan, Bromcyan und Dicyan, besonders bevorzugt sind Chlorcyan oder Dicyan.

In einer weiteren bevorzugten Ausführungform liegt das molare Verhältnis von Malonsäuredinitril zur Cyanoverbindung der Formel RCN im Bereich von 0,2 : 1 bis 2 : 1, besonders bevorzugt von 0,9 : 1 bis 1,1 : 1, ganz besonders bevorzugt bei 1 : 1.05 bis 1,05 : 1.

Geeignete Basen im erfindungsgemässen Verfahren können ausgewählt werden aus der Gruppe bestehend aus Alkali- und Erdalkalihydroxiden, Alkali- und Erdalkalicarbonaten, Ammoniak, primären, sekundären und tertiären Aminen. Besonders bevorzugt ist die Base ein Alkali- und Erdalkalihydroxid, ganz besonders bevorzugt Natriumhydroxid und/oder Kalimhydroxid.

In einem bevorzugten Verfahren wird der pH während der Reaktion bei einem Wert grösser 4 gehalten, vorzugsweise bei einem Wert grösser 7, besonders bevorzugt im Bereich von 7 bis 9.

Das Halogenid als Anion X⁻ⁿ des Salzes der Formel (Q⁺)ₙX⁻ⁿ kann ausgewählt werden aus der Gruppe bestehend aus Fluor, Chlor, Brom und Iod. Besonders bevorzugt ist es Chlorid.

Als Pseudohalgenidanionen X⁻ⁿ im Salz (Q⁺)ₙX⁻ⁿ können Anionen verwendet werden, die aus mindestens zwei elektronegativen Atomen bestehen und die den Halogenen chemisch ähnlich sind. Vorzugsweise werden die Pseudohalogenidanionen ausgewählt aus der Gruppe bestehend aus CN⁻, OCN⁻, SCN⁻ und N₃⁻. Besonders bevorzugt ist CN⁻.

Geeignete organische Säureanionen sind beispielsweise Anionen ein- und zweibasiger nichtaromatischer und aromatischer Säuren wie beispielsweise Acetat, Oleat, Fumerat, Maleat, Pyruvat, Oxalat und Benzoat. Besonders bevorzugt sind Acetat und Pyruvatanionen.

In einem bevorzugten Verfahren werden die gebildeten Tricyanomethansalze der Formel I, wobei Q⁺ wie oben definiert ist, von nicht umgesetzten Ausgangsverbindungen abgetrennt und durch Extraktionsverfahren gereinigt.

Die folgende Beispiele sollen die Erfindung verdeutlichen, ohne jedoch eine Einschränkung darzustellen.

### Beispiele:

### Beispiel 1: 1-Butyl-1-methylpyrrolidiniumchlorid

Methylpyrrolidin (150 g, 1,76 mol) wird vorgelegt und bei Raumtemperatur mit Butylchlorid (163 g, 1,76 mol) gemischt. Die erhaltene Reaktionsmischung wird dann für 24 h unter Rückfluss erhitzt (Reaktionstemperatur ca. 77 °C) wobei sich eine leicht gelbliche Suspension bildet. Danach wird die Reaktionsmischung auf Raumtemperatur gekühlt und filtriert. Der Rückstand wird mit wenig MTBE gewaschen und anschliessend bei 50 °C im Vakuum getrocknet und ergibt reines 1-Butyl-1-methylpyrrolidiniumchlorid (27 g, 0,09 mol, 8,6% Umsatz). Das restliche Material der Reaktionsmischung besteht ausschliesslich aus den nicht umgesetzten Startmaterialien Methylpyrrolidin und Butylchlorid, die vollständig zurückgewonnen werden. Der Schmelzpunkt des erhaltenen 1-Butyl-1-methylpyrrolidiniumchlorid beträgt 199,5 °C.
¹H-NMR (d₆-DMSO): δ = 3,50(m, 4H), 3,36 (m, 2H), 3,02 (s, 3H), 2,08 (s br, 4H), 1,68 (m, 2H), 1,33 (sextet, J = 7,3Hz, 2H) 0,94 (t, J = 7,6Hz, 3H);
¹³C-NMR (d₆-DMSO): δ = 64,0 (CH₂), 63,4 (CH₂), 48,1 (CH₃), 25,6 (CH₂), 21,8 (CH₂), 20,0 (CH₂), 14,2 (CH₃).

### Beispiel 2: 1-Butyl-1-methyl-pyrrolidiniumtricyanomethan

Malonsäuredinitril (17,8 g, 264 mmol) wird in Wasser (300 ml) vorgelegt und bei 0 bis 5 °C wird Chlorcyan (16,5 g, 0,268 mmol) in die Reaktionslösung eingeleitet, wobei der pH-Wert der Reaktionslösung während dem Einleiten auf 8,5 gehalten wird durch entsprechende Zugabe von 30%-iger Natronlauge. Die Reaktionstemperatur der Lösung wird während der Einleitung von Chlorcyan ca. 1 h auf 0 bis 5 °C gehalten. Zu der Reaktionsmischung wird eine Mischung aus 1-Butyl-1-methylpyrrolidiniumchlorid (47,5 g, 0,267 mmol) in Wasser (150 ml) zugegeben. Die organische Phase wird abgetrennt und die wässrige Phase mit Dichlormethan (2×100 ml) extrahiert. Die vereinigten organischen Phasen werden mit Wasser (4×75 ml) extrahiert. Die organische Phase wird mit Aktivkohle (3 g) versetzt und bei RT für 30 min gerührt. Anschliessend wird die organische Phase abfiltriert und am Rotationsverdampfer bei 40 bis 60 °C eingeengt (ca. 500 mbar bis ca. 20 mbar). Man erhält auf diese Weise 1-Butyl-1-methylpyrrolidiniumtricyanomethan (46.8 g, 201°mmol, Ausbeute 76 %. Der Chloridgehalt ist < 0,1 %. Die Viskosität bei 20 °C beträgt 33 mPas.
¹H-NMR (d₆-DMSO): δ = 3,44 (m, 4H), 3,28 (m, 2H), 2,98 (s, 3H), 2,08 (s br, 4H), 1,69 (m, 2H), 1,32 (sextet, J = 7,3Hz, 2H), 0,94 (t, J = 7,6Hz, 3H);
¹³C-NMR (d₆-DMSO): δ = 120,5 (C, q), 63,5 (CH₂), 63,0 (CH₂), 47,6 (CH₃), 24,9 (CH₂), 21,1 (CH₂), 19,3 (CH₂), 13,4 (CH₃), 4,7 (C, q).

### Beispiel 3: 1-Butyl-3-methyl-imidazoliumtricyanomethan

Eine Lösung aus 1-Butyl-3-methylimidazoliumchlorid (43,1 g, 178 mmol) in Wasser (100 ml) wird wie in Beispiel 2 beschrieben zu einer Mischung aus Malonsäuredinitril und Chlorcyan zugegeben. Man erhält so 1-Butyl-3-methylimidazoliumtricyanomethan (33,6 g, 147 mmol, 82% Ausbeute) mit einem Chloridgehalt von < 0,1 %. Die Viskosität bei 20 °C beträgt 30 mPAS.
¹H-NMR (d₆-DMSO): δ = 9,05 (s, 1H), 7,67 (t, J = 1,8Hz, 1H), 7,61 (t, J = 1,8Hz, 1H), 4,07, (t, J = 7,2Hz, 2H), 3,83 (s, 3H), 1,80 (t-hept, J¹ = 12,4Hz, J² =1,8Hz, 2H), 1,26, (t-hex, J¹ = 7,5Hz, J² =1,8Hz, 2H), 0,89 (t, J = 7,4Hz, 3H);
¹³C-NMR (d₆-DMSO): δ = 136,5 (CH), 123,5 (CH), 122,2 (CH), 120,5 (C, q), 48,6 (CH₂), 35,7 (CH₃), 31,3 (CH₂), 18,8 (CH₂), 13,1 (CH₃), 4,8 (C, q).

### Beispiel 4: 1-Ethyl-3-methyl-imidazoliumtricyanomethan

Eine Lösung aus 1-Ethyl-3-methylimidazoliumchlorid (178,4 g, 1,22 mol) in Wasser (180 ml) wird wie in Beispiel 2 beschrieben zu einer Mischung aus Malonsäuredinitril (84,6°g, 1,28°mol) und Chlorcyan (79,9°g, 1,30°mol) zugegeben. Die so erhaltene Reaktionsmischung ergibt keine Phasentrennung und wird zuerst mit Aktivkohle (3%) behandelt wie in Beispiel 2 und anschliessend mit Dichlormethan (300 ml) extrahiert. Die organische Phase wird dann mit Wasser (2×200 ml) gewaschen. Nach dem Einengen am Rotationsvedampfer bei 40 bis 60 °C (ca. 500 mbar bis ca. 20 mbar) werden 169,5 g (69% Ausbeute) 1-Ethyl-3-methyl-imidazoliumtricyanomethan erhalten mit einem Chlorid- und einem Natriumgehalt von jeweils weniger als 10 ppm.
¹H-NMR (d₆-DMSO): δ = 9,09 (s, 1H), 7,72 (t, J = 1,7 Hz, 1H), 7,64 (t, J = 1,7 Hz, 1H), 4,21 (t, J = 7,4Hz, 2H), 3,86 (s, 3H), 1,45, (t, J = 7,4Hz, 3H);
¹³C-NMR (d₆-DMSO): δ = 136,4 (CH), 124,4 (CH), 122,8 (CH), 121,7 (C, q), 45,4 (CH₂), 36,7 (CH₃), 15,8 (CH₃), 6,7 (C, q).

### Beispiel 5: 1-Butyl-3-methyl-pyridiniumtricyanomethan

Eine Lösung aus 1-Butyl-3-methylpyridiniumchlorid (939,6 g, 5,06 mol) in Wasser (813 ml) wird wie in Beispiel 2 beschrieben zu einer Mischung aus Malonsäuredinitril (351,0°g, 5,31°mol) und Chlorcyan (333,1°g, 5,42°mol) zugegeben. Die Reaktionsmischung trennt sich in zwei Phasen, wobei die wässrige Phase abgetrennt und mit Dichlormethan (1290 ml) extrahiert wird. Die organischen Phasen werden vereinigt, mit Aktivkohle (3%) behandelt und dann mit Wasser (4×900 ml) gewaschen. Nach dem Einengen am Rotationsverdampfer bei 40 bis 60 °C (ca. 500 mbar bis ca. 20 mbar) werden 1118 g (92% Ausbeute) 1-Butyl-3-methylpyridiniumtricyanomethan erhalten mit einem Chlorid- und einem Natriumgehalt von jeweils weniger als 10 ppm.
¹H-NMR (d₆-DMSO): δ = 8,95 (s, 1H), 8,87 (d, J = 6,1Hz, 1H), 8,40 (d, J = 8,0Hz, 1H), 8,01 (t, J = 7,1 Hz, 1H), 4,53, (t, J = 7,4Hz, 2H), 2,49 (s, 3H), 1,90 (m, 2H), 1,29, (t-hex, J = 7,5Hz, 2H), 0,90 (t, J = 7,4Hz, 3H);
¹³C-NMR (d₆-DMSO): δ = 145,5 (CH), 144,1 (CH), 141,8 (CH), 138,7 (C, q), 127,2 (CH), 120,4 (C, q), 60,4 (CH₂), 32,4 (CH₂), 18,7 (CH₂), 17,7 (CH₃), 13,10 (CH₃), 4,6 (C, q).

### Beispiel 6: 1-Oktyl-3-methyl-pyridiniumtricyanomethan

Eine Lösung aus 1-Oktyl-3-methylpyridiniumchlorid (125,8 g, 520°mmol) in Wasser (300 ml) wird wie in Beispiel 2 beschrieben zu einer Mischung aus Malonsäuredinitril (36,0°g, 534°mmol) und Chlorcyan (31,4°g, 537°mmol) zugegeben. Die Reaktionsmischung trennt sich in zwei Phasen, wobei die wässrige Phase abgetrennt und mit Dichlormethan (300°ml) extrahiert wird. Die organischen Phasen werden vereinigt, mit Aktivkohle (3%) behandelt und dann mit Wasser (4×125 ml) gewaschen. Nach dem Einengen am Rotationsverdampfer bei 40 bis 60 °C (ca. 500 mbar bis ca. 20 mbar) erhält man 137,8 g (89% Ausbeute) 1-Oktyl-3-methylpyridiniumtricyanomethan mit einem Chlorid- und einem Natriumgehalt von jeweils weniger als 100 ppm.
¹H-NMR (d₆-DMSO): δ = 8,96 (s, 1H), 8,88 (d, J = 6,3Hz, 1H), 8,40 (d, J = 8,0Hz, 1H), 8,01 (dd, J¹ = 8.2Hz, J²=6,2Hz, 1H), 4,52 (t, J = 7,4Hz, 2H), 2,49 (s, 3H), 1,90 (p, J = 7,1Hz, 2H), 1,26 (m, 10H), 0,83 (m, 3H);
¹³C-NMR (d₆-DMSO): δ = 145,5 (CH), 144,0 (CH), 141,8 (CH), 138,7 (C, q), 127,2 (CH), 120,3 (C, q), 60,6 (CH₂), 31,0 (CH₂), 30,5 (CH₂), 28,3 (CH₂), 28,2 (CH₂), 25,3 (CH₂), 21,9 (CH₂), 17,7 (CH₃), 13,7 (CH₃), 4,6 (C, q).

## Patentansprüche

1. Verbindungen der Formel 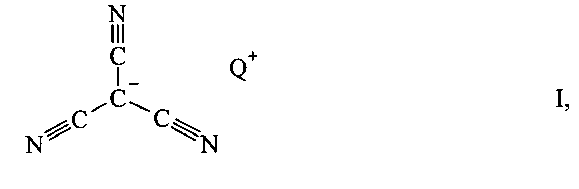
worin Q⁺ ein organisches Kation ist, das ein mindestens ein Heteroatom enthält, ausgewählt aus der Gruppe bestehend aus Stickstoff, Phosphor, Schwefel und Sauerstoff.

2. Verbindungen nach Anspruch 1, worin das organische Kation Q⁺ ausgewählt ist aus der Gruppe bestehend aus Kationen der Formel
a) (WR¹R²R³R⁴)⁺, worin W ein Stickstoff- oder Phosphoratom bedeutet, und
i) worin R¹ bis R³ unabhängig voneinander C₁₋₂₀-Alkyl sind, und R⁴ C₁₋₂₀-Alkyl, C₃₋₁₀-Cycloalkyl oder C₆₋₁₀-Aryl ist, wobei R¹ bis R⁴ unabhängig voneinander gegebenenfalls ein oder mehrere Halogenatome enthalten, oder
ii) worin R¹ und R² zusammen einen 5- bis 7-gliedrigen Ring bilden, und worin R³ und R⁴ unabhängig voneinander C₁₋₂₀-Alkyl sind, wobei R³ und R⁴ unabhängig voneinander gegebenenfalls ein oder mehrere Halogenatome enthalten, oder
iii) worin R¹ und R², sowie R³ und R⁴ jeweils einen 5- bis 7-gliedrigen Ring bilden, oder
b) (XR⁵R⁶R⁷)⁺, worin X ein Stickstoffatom bedeutet, und worin R⁵ und R⁶ gemeinsam mit X einen Ring bilden, in welchem X formal eine Einfach- und eine Doppelbindung zu R⁵ und R⁶ aufweist, und R⁷ C₁₋₂₀-Alkyl, C₃₋₁₀-Cycloalkyl oder C₆₋₁₀-Aryl ist, wobei R⁷ gegebenenfalls ein oder mehrere Halogenatome enthält, oder
c) (YR⁸R⁹R¹⁰)⁺, worin Y ein Schwefelatom bedeutet, und
i) worin R⁸ und R⁹ unabhängig voneinander C₁₋₂₀-Alkyl sind, und R¹⁰ C₁₋₂₀-Alkyl, C₃₋₁₀-Cycloalkyl oder C₆₋₁₀-Aryl ist, wobei R⁸ bis R¹⁰ unabhängig voneinander gegebenenfalls ein oder mehrere Halogenatome enthalten, oder
ii) worin R⁸ und R⁹ gemeinsam einen 5- bis 7-gliedrigen Ring bilden, und R¹⁰ C₁₋₂₀-Alkyl, C₃₋₁₀-Cycloalkyl oder C₆₋₁₀-Aryl ist, wobei R¹⁰ gegebenenfalls ein oder mehrere Halogenatome enthält, oder
d) (ZR¹¹R¹²)⁺, worin Z ein Sauerstoff- oder Schwefelatom bedeutet, und worin R¹¹ und R¹² gemeinsam mit Z einen Ring bilden, in welchem Z formal eine Einfach- und eine Doppelbindung zu R¹¹ und R¹² aufweist, und
worin an jeden der von den Substituenten R¹ bis R¹² gebildeten Ringe gegebenenfalls ein oder mehrere Substituenten aus der Gruppe bestehend aus C₁₋₂₀-Alkylgruppen, C₁₋₂₀-Alkoxygruppen, C₃₋₁₀-Cycloalkylgruppen, C₆₋₁₀-Arylgruppen, Halogen und Cyan gebunden sind, wobei die C₁₋₂₀-Alkyl-, C₁₋₂₀-Alkoxy-, C₃₋₁₀-Cycloalkyl- und C₆₋₁₀-Arylgruppen unabhängig voneinander gegebenenfalls ein oder mehrere Halogenatome enthalten, und
worin an jeden der von den Substituenten R¹ bis R¹² gebildeten Ringe gegebenenfalls ein oder zwei weitere Heteroatome aus der Gruppe bestehend Stickstoff, Schwefel und Sauerstoff enthalten, und/oder an einen anderen aromatischen oder nichtaromatischen 5- bis 7-gliedrigen Ring anelliert ist.

3. Verbindungen nach Anspruch 1 oder 2, worin das Kation Q⁺ ausgewählt ist aus der Gruppe bestehend aus organischen Ammonium-, Phosphonium- und Sulfoniumkationen, Pyrrolidinium-, Pyrrolinium-, Pyrazolium-, Pyrazolium, Imidazolium-, Triazolium-, Oxazolium-, Thiazolium-, Piperidinium-, Piperazinium-, Morpholinium-, Pyridinium-, Pyridazinium-, Pyrimidinium-, Pyrazinium-, 1,3-Dioxolium-, Pyrilium-, Thiopyrilium-, Azoniaspiro- und Phosphoniaspirokationen.

4. Verwendung der Verbindungen gemäss den Ansprüchen 1 bis 3, gegebenenfalls in einer Mischung mit einer oder mehreren anderen ionischen Flüssigkeiten, Wasser oder organischen Lösungsmitteln, als polare aprotische Lösungsmittel.

5. Verfahren zur Herstellung der Tricyanomethansalze gemäss den Ansprüchen 1 bis 3, umfassend:
a) Mischen von Malonsäuredinitril mit einer Cyanoverbindung der Formel RCN, worin R ausgewählt ist aus der Gruppe bestehend aus Chlor, Brom, Iod und Cyan in Gegenwart einer Base, gegebenenfalls in Gegenwart eines Lösungsmittels,
b) Zugabe eines Salzes (Q⁺)ₙXⁿ⁻ mit n = 1 oder 2, wobei Q⁺ wie oben definiert, und Xⁿ⁻ ein Anion ausgewählt aus der Gruppe bestehend aus Halogeniden, Pseudohalogeniden, Sulfat und organischen Säureanionen ist,
wobei das Anion Xⁿ⁻ in wässriger Phase gegen ein aus Malonsäuredinitril und RCN *in situ* gebildetes Tricyanomethanidanion ausgetauscht wird.

6. Verfahren nach Anspruch 5, worin Malonsäuredinitril, die Cyanoverbindung RCN und das Salz (Q⁺)ₙXⁿ⁻ in Gegenwart einer Base, gegebenenfalls in Gegenwart eines weiteren Lösungsmittels, in wässriger Phase gemischt werden und wobei Bildung und Austausch des Tricyanomethanidanions in einer Eintopfreaktion stattfinden.

7. Verfahren nach Anspruch 5 oder 6, worin das molare Verhältnis von Malonsäure dinitril zur Cyanoverbindung RCN im Bereich von 0,2 : 1 bis 2 : 1 liegt, besonders bevorzugt von 0,9 : 1 bis 1,1 : 1, ganz besonders bevorzugt bei 1 : 1.05 bis 1,05 : 1.

8. Verfahren nach einem der Ansprüche 5 bis 7, worin die Base ausgewählt ist aus der Gruppe bestehend aus Alkali- und Erdalkalihydroxiden, Alkali- und Erdalkalicarbonaten, Ammoniak, primären, sekundären und tertiären Aminen, Pyridinen und Imidazolen.

9. Verfahren nach einem der Ansprüche 5 bis 8, worin der pH während der Reaktion bei einem Wert grösser 4 gehalten wird, vorzugsweise bei einem Wert grösser 7, besonders bevorzugt im Bereich von 7 bis 9.

10. Verfahren nach einem der Ansprüche 5 bis 9, worin das Anion Xⁿ⁻ ausgewählt wird aus der Gruppe bestehend aus Chlorid, Bromid und Cyanid.
